# EUROPEAN PATENT APPLICATION

(11) **EP 1 500 655 A1**
(43) Date of publication of application: **26.01.2005**
(21) Application number: 03711802.3
(22) Date of filing: 25.03.2003
(51) Int. Cl.: C07D 277/22, C07D 277/38, C07D 263/30, C07D 263/48, C07D 207/30, C07D 207/34, C07D 401/12, C07D 403/12, C07D 405/12, C07D 407/12, C07D 417/12, A61K 31/40, A61K 31/41

(54) **NEW METHIONINE AMINOPEPTIDASE INHIBITOR**

(30) Priority: 02.04.2002 CN 02111230
(71) Applicant: Shanghai Institute of Materia Medica, Chinese Academy of Sciences, Pudong New, Shanghai 201203 (CN)
(72) Inventor: NAN, Fajun, The National Center For Drug Screening, Pudong New, Shanghai 201203 (CN); YE, Qizhuang, The National Center For Drug Screen., Pudong New, Shanghai 201203 (CN); LI, Jingya, The National Center For Drug Screening, Pudong New, Shanghai 201203 (CN); LIU, Zhiying, The National Center For Drug Screen., Pudong New, Shanghai 201203 (CN); LUO, Qunli, The National Center For Drug Screening, Pudong New, Shanghai 201203 (CN); CUI, Yongmei, The National Center For Drug Screen., Pudong New, Shanghai 201203 (CN)
(74) Representative: Epping Hermann & Fischer
(86) International application number: PCT/CN2003/000213
(87) International publication number: WO 2003/082838

(57) **Abstract**

The invention provides a new methionine aminopeptidase inhibitors with the following formula (I), wherein R₁, R₂, R₃, R₄, R₅, R₆ and X have the meanings given in the description. Pharmacological experiment shows that they display good inhibition activity for methionine aminopeptidase.

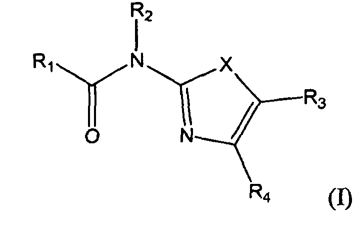

## Description

### FIELD OF THE INVENTION

The present invention relates to a series of small-molecular organic compounds having potent biological activity for inhibiting methionine aminopeptidases (MetAPs), also they showed certain selectivity for different subtypes of MetAPs, therefore they may be used as a new type of antitumor and antibacterial lead compounds.

### BACKGROUND OF THE INVENTION

Proteins synthesized in eukaryotic cells are initiated with an N-terminal methionine residue, and proteins synthesized in prokaryotes, mitochondria, and chloroplasts are initiated with an N-terminal formylmethionine residue. The formyl group is removed by a peptide deformylase before methionine aminopeptidases (MetAPs) remove the N-terminal methionine in a non-processive manner. MetAPs represent a unique class of proteases that are capable of removing the N-terminal methionine residues from nascent polypeptide chains. Removal of the initiator methionine residue is often required for posttranslational modifications to the N-terminus, such as myristoylation and acetylation, which lead to proper function, localization and stability of proteins.

*E. coli* methionine aminopeptidase was firstly observed as a member of MetAPs family, later, people also found analogous enzymes in *S. cerevisiae* and mammalian with similar structure and function. Comparison of sequence reveals that despite this type of MetAPs has high homology in the C-terminal domains, its N-terminal domain has an additional sequence like zinc finger motif. These extensions may be involved in the association of the enzyme with the ribosome and have no effect on its activity *(J. Biol. Chem.* 1990; *265:* 19892-19897). This type enzyme and *E. coli* methionine aminopeptidase were classified as type I enzyme; Another type of methionine aminopeptidase were later isolated and cloned from porcine liver *(J.Biol.Chem.1992;* 267: 20667-20673). Comparison of the structure shows that its C-terminal domain, with an insertion of approximately 60 residues, has lower overall sequence homology with *E. coli* methionine aminopeptidase than type I enzyme; also it has a highly charged N-terminus with alternating polyacidic and polybasic residues instead of zinc finger motif, this part of structure has proven to be the inhibitor of phosphorylation of the eukaryotic initiation factor (eIF-2α), with no effect on activity of the enzyme. In order to be differentiated from type I enzyme, it was defined type II enzyme, such as *S. cerevisiae,* drosophila, mice, and human type *II* enzyme being found subsequently.

Methionine aminopeptidases (MetAPs) exist in the cells of all organisms and have shown remarkable specificity for the substrates. The experiments of endogenetic proteins, recombinant proteins in vitro or polypeptides as substrates *(Biochemistry.* 1987; 26:8242-8246), showed that the terminal methionine of the protein or polypeptide substrate is often removed if it is followed by one of the small, uncharged amino acid residues with the side chain length <3.68 Å. Usually, it is believed that any one of the following seven amino acids including glycine, alanine, serine, threonine, proline, valine, and cysteine in the penultimate position direct MetAPs to cleave the initiator methionine *(J. Bacteriol. 1987; 169:* 751-757; *Biochemistry.* 1999; 38: 14810-14819).

MetAPs represent a unique class of proteases that are capable of removing the N-terminal methionine residues from nascent polypeptide chains. Removal of the initiator methionine residue is often required for proper localization, targeting, and eventual degradation of proteins *(J.Biol. Chem.1982; 257:* 3532-3536).

The MetAPs inhibitors can be divided into covalent and non-covalent binding inhibitors according to the mode of the action. Moreover, there is another type of inhibitors including reaction product and transition-state analogues, the interactions with which are very similar to those seen for the enzyme with a bestatin-based inhibitor *(Biochemistry.1999; 38:* 14810-14819).

The compounds binding to MetAPs covalently such as fumagillin and its synthetic analog TNP-470 (with IC₅₀ in the nM range) compose a class of structurally related natural products that potently inhibit angiogenesis by blocking endothelial cell proliferation. A common target for fumagillin and TNP-470 was identified as the type 2 methionine aminopeptidase (MetAPs). These compounds prove to inactivate the enzyme by covalent modification of a histidine residue in the active site *(Proc.Natl.Acad.Sci. USA*.1998; *95:* 15183-15188).

Non-covalent inhibitors have been designed from a potent inhibitor of leucine aminopeptidase, Bestatin, according to the characteristics of MetAPs substrates. In modifying it for MetAPs, its chain was replaced with that of norleucine and an alanine was placed at the P1' position. The resulting non-hydrolyzable substrate analog AHHPA-Ala-Leu-Val-Phe-OMe inhibited *Ec*MetAP1 with an IC₅₀ of 5 µM, and was the best inhibitor of *Ec*MetAP 1 reported up to now. 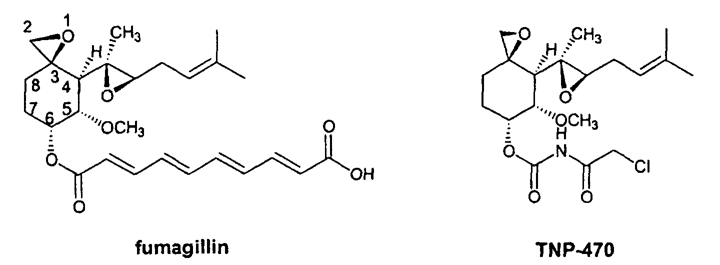 The covalent-binding inhibitors of MetAPs 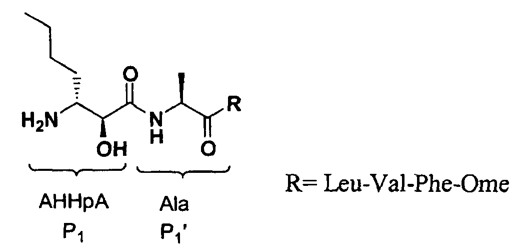 The non-covalent inhibitor of *Ec*MetAP 1

Angiogenesis is the process by which new blood vessels are grown, and is essential for reproduction, development and repair. Angiogenesis becomes pathogenic as the growth of blood vessels intensifies. New blood vessels are required for tumor growth; therefore, insight into biochemical mechanisms which regulate angiogenesis will aid in the diagnosis and treatment of cancer. Angiogenesis is a critical component of tumor metastasis, providing an avenue for tumor cells to leave the primary site and enter the blood stream. Cancer cells cannot grow or spread without the formation of new blood vessels. An increase in newly formed, highly permeable blood vessels surrounding a tumor serves to provide oxygen and essential nutrients to the tumor as well as to increase the potential for tumor cells to be released into circulation. So inhibition of angiogenesis has become a topic of much interest in cancer research. Fumagillin and its derivatives have been shown to block angiogenesis both in vitro and in vivo models by their inhibition of endothelial cell proliferation and display inhibitory activity in animal models of tumor growth, which support the potential of MetAP2 inhibition as an approach to the treatment of cancer and other diseases with an angiogenic component.

The presence of MetAPs is essential for cell viability, and disruption of the gene for MetAP in *E. coli* (*Ec*MetAP1) or *Salmonella typhimurium* is a lethal event, disruption of either gene of yeast *Saccharomyces cerevisiae* resulted in a slow-growth phenotype. Therefore, the MetAP enzymes present good targets for new antibiotic drug discovery, and inhibitors against MetAPs offer hope for a new treatment of bacterial and fungal infections.

### SUMMARY OF THE INVENTION

Aim of the invention: The invention provides a series of small molecular methionine aminopeptidase inhibitors with a novel structure and their structure-activity relationship. These compounds may be used to reveal the function of MetAPs in physiologica and pathologicall conditions, and also used as antitumor, antibacterial and anti-infection lead compounds.

The invention also provides the preparation of these methionine aminopeptidase inhibitors.

In its principle embodiment, the present invention provides a new type of methionine aminopeptidase inhibitors having the generalized structure: 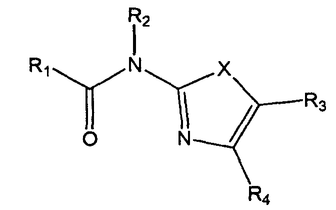 wherein
R₁ is selected from the group consisting of C₁-C₄ alkyl, substituted alkyl, C₃-C₆ cycloalkyl, substituted cycloalkyl, aryl, pyridyl; substituted aryl and substituted pyridyl wherein the substituents can be optionally selected from the group consisting of C₁-C₄ alkyl, nitro, carboxyl, aldehyde, alkoxy, alkylamino, acylamide, alkylthio; heterocycle or substituted heterocycle having the following structure: 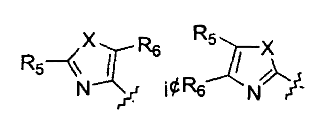 R₅, R₆ are selected from the group consisting of hydrogen, C₁-C₄ alkyl, substituted alkyl, C₃-C₆ cycloalkyl, substituted cycloalkyl, aryl, pyridyl; substituted aryl and substituted pyridyl wherein the substituents can be optionally selected from the group consisting of nitro, carboxyl, aldehyde, alkoxy, alkylamino, acylamide, alkylthio;
R₂ is selected from the group consisting of hydrogen, C₁-C₄ alkyl, substituted alkyl, aryl, substituted aryl wherein the substituents can be optionally selected from the group consisting of C₁-C₄ alkyl, nitro, carboxyl, aldehyde, alkoxy, alkylamino, acylamide, alkylthio;
R₃ is selected from the group consisting of hydrogen, C₁-C₄ alkyl, substituted C₁-C₄ alkyl, halogen atoms; aryl, substituted aryl;
R₄ is selected from the group consisting of hydrogen, C₁-C₄ alkyl, substituted alkyl, substituted aryl;
X is selected from the group consisting of O, S, N.

The invention also provides the preparation of these methionine aminopeptidase inhibitors as the following formula:

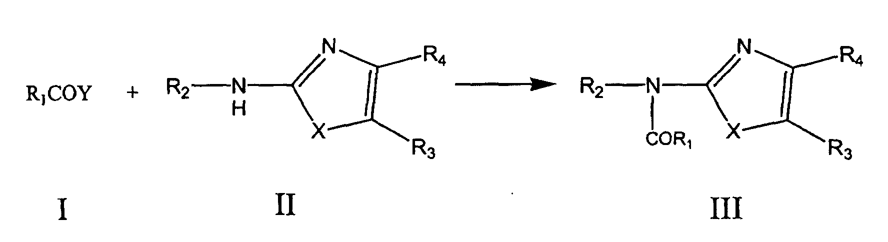

The syntheses of compounds III could be accomplished by the well-precedented condensation of compound *I*(wherein Y is selected from OH, Cl or other activated group) with compound II, the solvent used in this reaction may be CH₂Cl₂, DMF, CH₂ClCH₂Cl, toluene, benzene, H₂O, dioxane or the mixed solvent when needed such as CH₂Cl₂/DMF (1:1 V/V). The dehydration reagents used in this reaction may be DCC, ECD, DIC, HBTU according to the properties of the compounds, in some cases, the proper activated reagents were used, such as HOBT, pentafluorophenol, molecular sieves, in some cases, the proper base such as Et₃N, I-Pr₂EtN, Pyridine, DMAP were used as catalysts, the reaction temperature is from -20°C to room temperature, in some cases, heating is necessary, from 50° C to 130°C. The reaction time is determined by the activated group of the reactants, when Y is Cl, the reaction is over in minutes, and some reactions need longer time, usually TLC monitoring was used to determine the process of the reaction. When the reaction is completed, the mixture was generally extracted with EtOAc, CH₂Cl₂ or CHCl₃. After the mixture was washed with 5% aqueous HCl, water and saturated aqueous NaCl, the combined organic phases were dried, and then concentrated under reduced pressure at low temperature and chromatographed to give the desired compound *III.* The reaction yield is changed according to the properties of reactants *I*and II, from 20% to 95%, and the obtained products were proved by NMR etc.

Compound II was made as reported in J. Org. Chem. 63, 196-200 (1998).

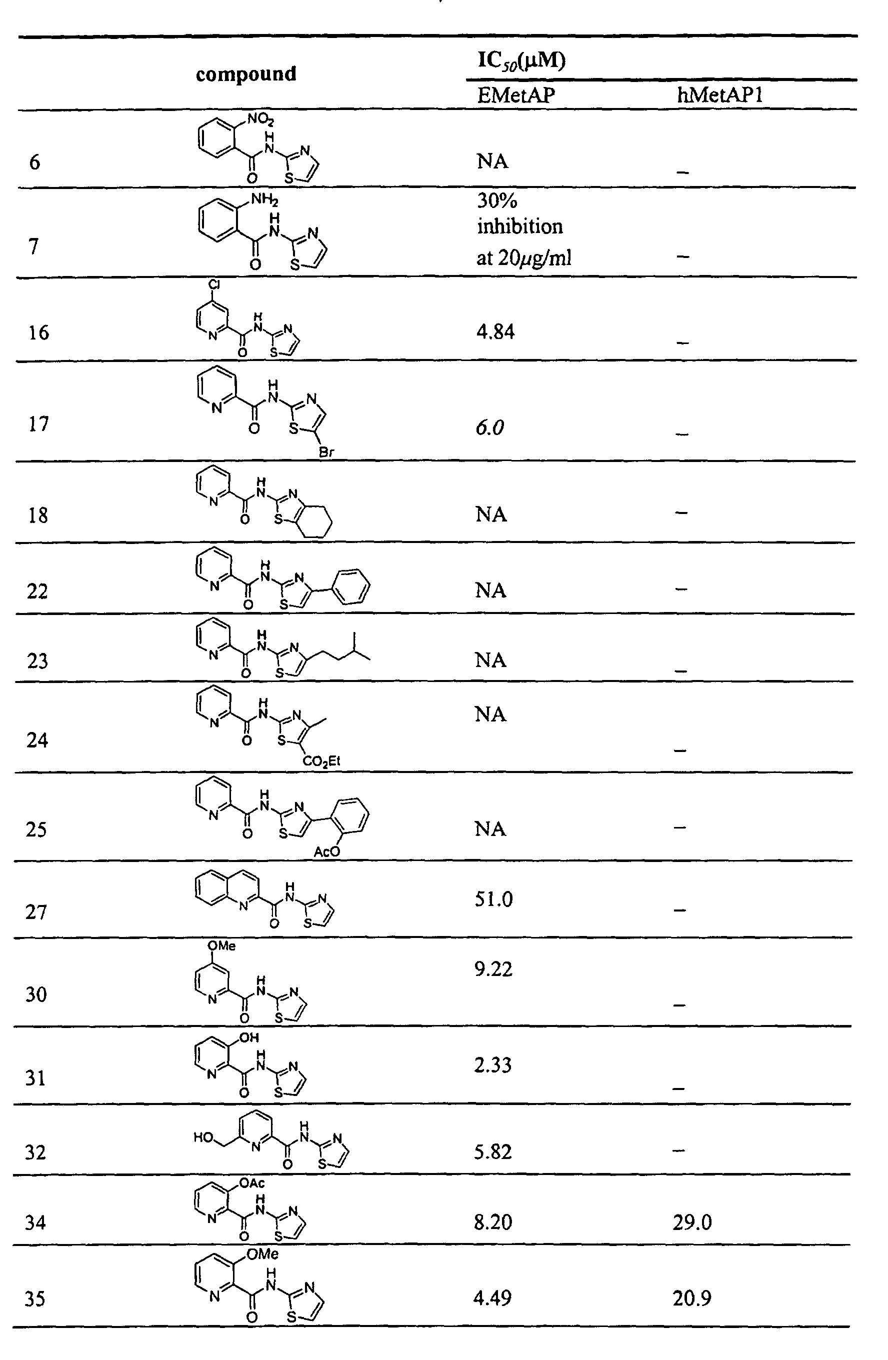

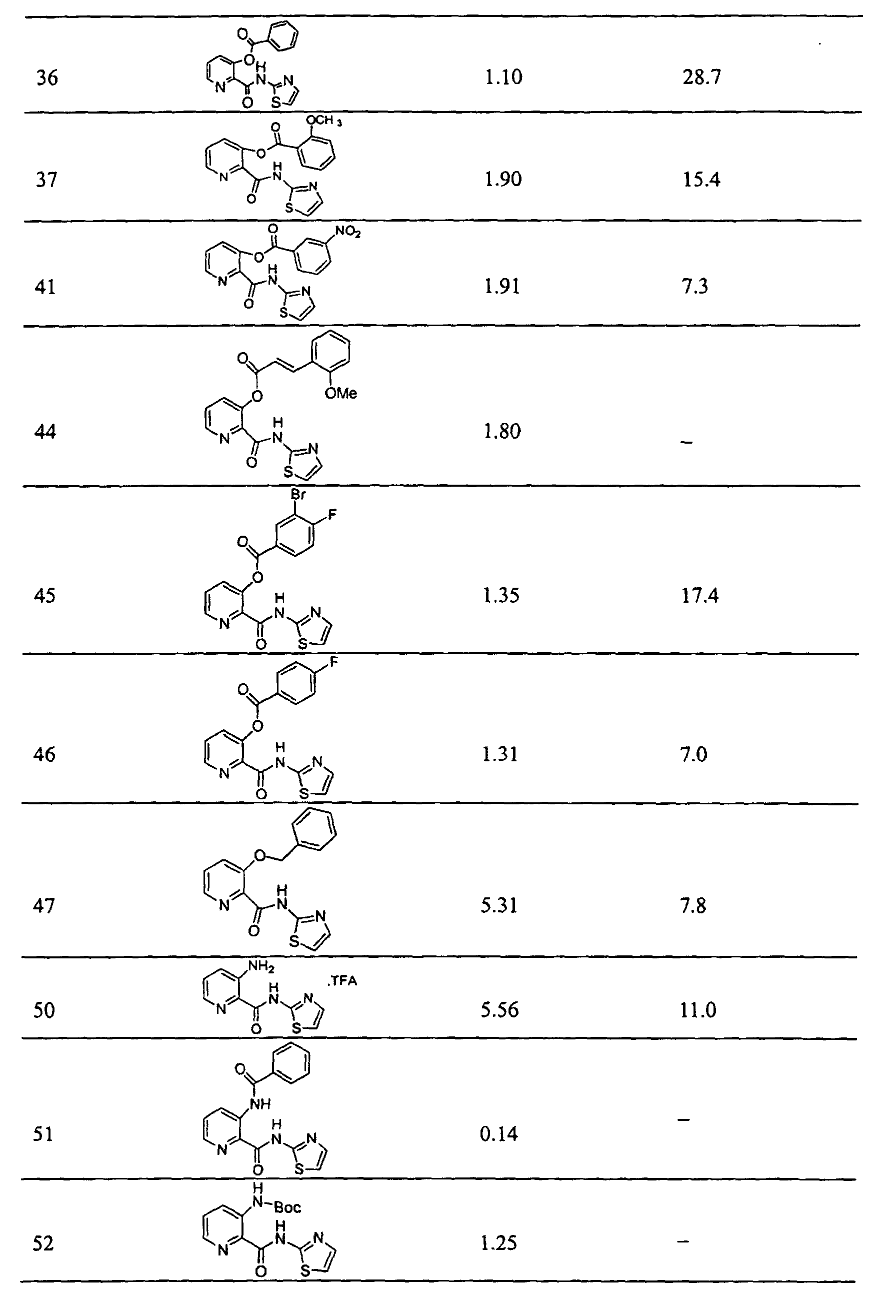

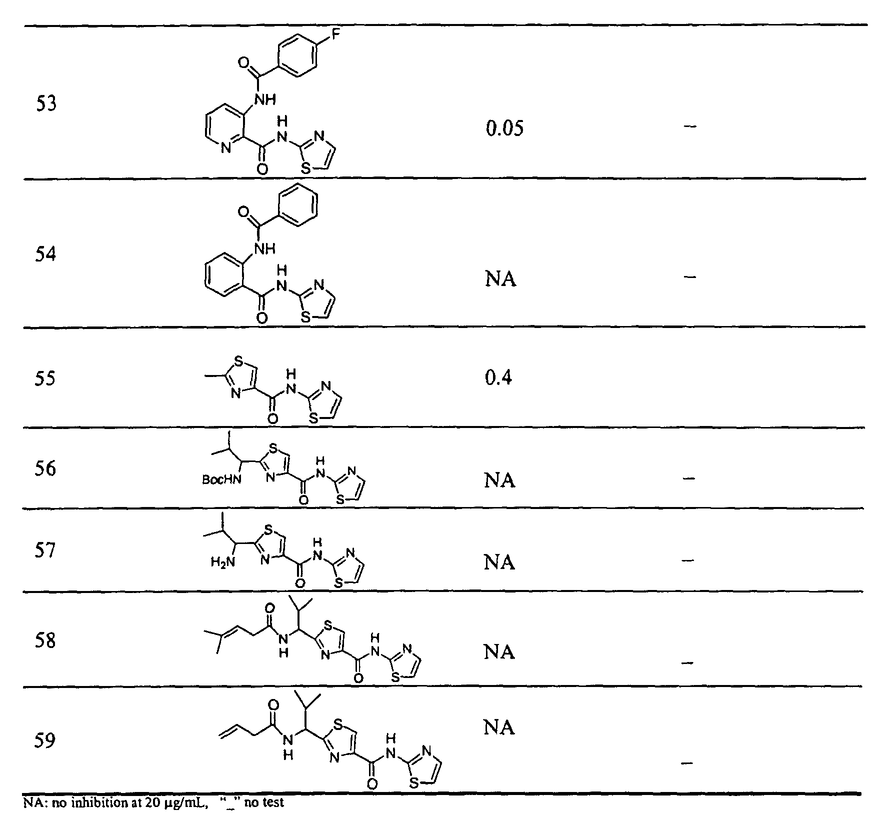

### Bioactivity Assay

*Ec*MetAP was cloned and overexpressed in *E. coli,* and purified by (NH₄)₂SO₄ precipitation and Q-Sepharose column separation to give apo-*Ec*MetAP. Finally the apo-*Ec*MetAP was incubated with Co (⊇) ions at suitable concentration to give the highly active enzyme, which can be used in screening enzyme inhibitors.

### Assay Principles of Drug Screening Model

Hydrolysis of the synthesized Met-S-C-Phe thiopeptolide substrate generates a free thiol group, which reacts with excessive DTNB instantly to produce a highly chromogenic product, 3-carboxyl-4-nitrothiophenol (ε₄₁₂ = 13,600 M⁻¹ cm⁻¹). Therefore, the enzymatic reaction catalyzed by MetAP can be continuously monitored at 412nm on a SpectraMAX 340 microplate reader.

### Operating Procedure

Employed conventional screening method (Anal Biochem. 2000, 280: 159-65)

All the compounds were initially screened at the multiple concentrations 2µg/mL, 20µg/mL, 100µg/mL. When the inhibitory activities of the compounds were larger than 50%, their estimated IC₅₀ value were determinated at eight inhibitor concentrations.

### Advantages of the invention

The compounds synthesized in the invention were a series of potent MetAP inhibitors with novel structures; Compared with the known enzyme inhibitors, their structures were relatively simple and easy to prepare. Moreover, some of them were the most efficient *Ec*MetAP inhibitors reported to date.

### EXEMPLIFICATION

The invention now being generally described, it will be more readily understood by reference to the following examples, which are included merely for purpose of illustration of certain aspects and embodiments of the present invention, and are not intended to limit the invention.

¹H-NMR spectra were recorded on a Varian MercuryAMX 300 spectrometer. Mass spectra (EI, 70eV except for additional note) were obtained on a VG ZAB-HS or VG-7070 spectrometer. All solvents were distilled prior to use and purified by standard methods. All reactions were carried out under an atmosphere of dry argon and checked by TLC unless otherwise stated, work up of the reactions was always washed with brine and dried over anhydrous MgSO₄. The products were purified by silica gel (200-300 mesh) chromatography unless otherwise mentioned. Silica gel including200-300mesh and GF₂₅₄ were produced by Qigndao Haiyang Chemical Co., Ltd., or Yantai Yuanbo silica Gel Co., Ltd..

### 1. Preparation of compound 6

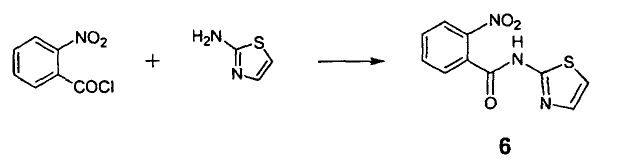

To a stirred solution of 2-nitro-benzoyl chloride (5 mmol) in CH₂Cl₂ (15 mL) was added thiazole-2-ylamine (500 mg, 5 mmol) and Et₃N (0.75 mL, 5 mmol). The reaction mixture was stirred at room temperature for 8h, and then totally transferred to the separate funnel and washed with 5% aqueous HCl. Some undissolved solid floated in the separate interface of the two phases. The solid was washed with CH₂Cl₂ and chromatographed (3:1 PE/EtOAc V/V) to yield 6 (503 mg, 40.4%) as a white solid. ¹H NMR (DMSO, 300 MHz): δ (ppm) 8.18 (d, *J*= 8.1 Hz, 1H), 7.91-7.86 (m, 1H), 7.82-7.77 (m, 2H), 7.55 (d, *J*= 3.6 Hz, 1H), 7.34 (d, *J*= 3.6 Hz, 1H).

### 2. Preparation of compound 18

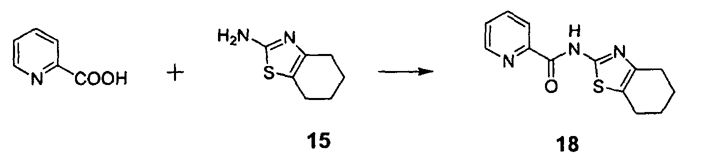

A mixture of 15 (195 mg, 1.26 mmol), pyridine-2-carboxylic acid (156 mg, 1.26 mmol), DCC (270 mg, 1.30 mmol), DMAP (11mg, cat) in CH₂Cl₂ (5 mL) was stirred at room temperature for 8h under argon atmosphere. The mixture was diluted with EtOAc and filtered. The filtrate was concentrated under reduced pressure. Chromatography (4:1, PE/EtOAc V/V) afforded 247 mg (75.7 %) of 18 as a white solid.
¹H NMR (CDCl₃, 300 MHz): δ (ppm) 11.08 (s, 1H), 8.62 (d, *J =* 5.2 Hz, 1H), 8.27 (d, *J =* 7.8 Hz, 1H), 7.92 (dt, *J* = 7.8,7.8,1.5 Hz, 1H), 7.51 (dd, *J* = 7.8, 5.2 Hz, 1H), 2.72 (d, *J* = 13.5 Hz, 4H), 1.88 (s, 4H);
¹³C NMR (CDCl₃, 300 MHz): δ (ppm) 161.76 (1C), 154.61 (1C), 148.76 (1CH), 148.18 (1C), 145.17 (1C), 137.90(1CH), 127.33(1CH), 123.59 (1C), 122.91 (1CH), 26.65 (1CH₂), 23.56 (1CH₂), 23.23 (2CH₂).

### 3. Preparation of compound 19

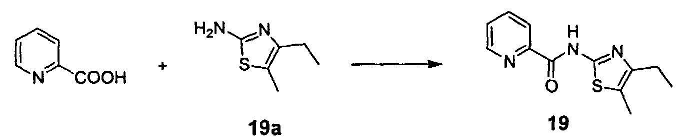

A mixture of 19a (28 mg, 0.197 mmol), pyridine-2-carboxylic acid (25 mg, 0.197 mmol), DCC (43 mg, 0.20 mmol), DMAP (cat) in CH₂Cl₂ (1mL) was stirred at room temperature for 8h under argon atmosphere. The mixture was diluted with EtOAc and filtered. The filtrate was concentrated under reduced pressure. Chromatography (5:1, PE/EtOAc VN) afforded 14 mg (75.7 %) of 19 as a white solid.
¹H NMR (CDCl₃, 300MHz): δ (ppm) 11.02 (s, 1H), 8.61 (dd, *J* = 4.8,1.5 Hz, 1H), 8.27 (d, *J* = 7.5 Hz, 1H), 7.91(dt, *J* = 7.5,7.5,1.5 Hz, 1H), 7.50 (ddd, *J* = 7.5,4.8,1.5 Hz, 1H), 2.62 (q, *J*= 7.5,7.5,7.5 Hz, 2H), 2.34 (s, 3H), 1.22 (t, *J=* 7.5,7.5 Hz, 3H).

### 4. Preparation of compound 22

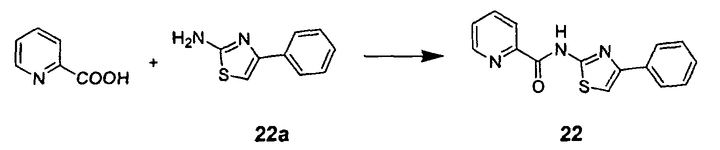

A mixture of 22a (23 mg, 0.131 mmol), pyridine-2-carboxylic acid (17 mg, 0.131 mmol), DCC (29 mg, 0.14 mmol), DMAP (cat) in CH₂Cl₂ (2 mL) was stirred at room temperature for 8h under argon atmosphere. The mixture was diluted with EtOAc and filtered. The filtrate was concentrated under reduced pressure. Chromatography (5:1, PE/EtOAc V/V) afforded 21 mg (75.7%) of 22 as a cyan solid.
¹H NMR (CDCl₃, 300MHz): δ (ppm) 11.25(s, 1H), 8.66 (d, *J=* 4.5Hz, 1H), 8.30 (d, *J* = 7.8Hz, 1H), 7.92 (dd, *J* = 7.8,7.8Hz, 1H), 7.89 (d, *J* = 7.8Hz, 2H), 7.53 (dd, *J =* 7.8,4.5Hz, 1H), 7.43 (t, *J*= 7.8,7.8Hz, 2H), 7.33 (t, *J*= 7.8,7.8Hz, 1H), 7.22 (s, 1H); ¹³C NMR (CDCl₃, 300MHz): δ (ppm) 162.34 (1C), 157.45 (1C), 150.73 (1C), 148.86 (1CH), 147.98 (1C), 138.02 (1CH), 134.63(1C), 128.98(2CH), 128.27 (1CH), 127.59 (1CH), 126.34 (2CH), 123.08 (1CH), 108.21 (1CH).

### 5. Preparation of compound 36

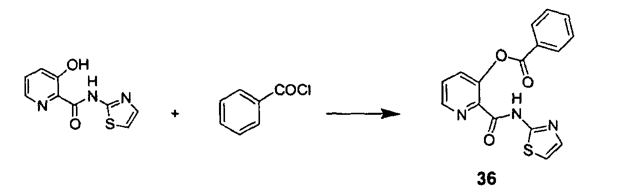

To a solution of 33 (20 mg, 0.09 mmol) in 4 mL of CH₂Cl₂ was added Et₃N (0.1 mL). The mixture was cooled to -78°*C*, and then benzoyl chloride (0.13 mmol, 1.5 eq) was added. The reaction mixture was stirred at -78°*C* for 3h, and then allowed to warm to room temperature and stirred for 2h. The solvent was evaporated and the residue was chromatographed on silica gel with PE/EtOAc (3:1) to give compound 36.
¹H NMR (CDCl₃, 300MHz): δ (ppm) 11.33 (br, 1H), 8.60 (d, *J* = 4.5 Hz, 1H), 8.29 (d, *J*= 7.5 Hz, 2H), 7.75-7.63 (m, 3H), 7.56 (t, *J=* 4.5,4.5 Hz, 2H), 7.50 (d, *J=* 3.0 Hz, 1H), 7.05 (d, *J* = 3.0 Hz, 1H);
¹³C NMR (CDCl₃, 300MHz): δ (ppm) 165.13 (1C), 160.29 (1C), 157.82 (1C), 148.59 (1C), 146.03 (1CH), 139.88 (1C), 137.87 (1CH), 134.12 (1CH), 133.90 (1CH), 130.85 (2CH), 129.13 (1C), 128.88 (2CH), 128.74 (1CH), 113.83 (1CH);
EIMS (m/z): 325 (M⁺, 7%), 226 (8), 197 (6), 127 (16), 105 (69), 97 (53), 91 (69), 85 (63), 71 (100), 69 (88).

### 7. Preparation of compound 44

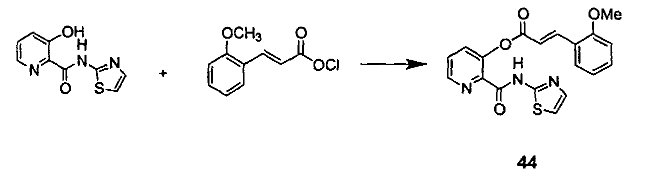

To a solution of 33 (98 mg, 0.45 mmol) in 8 mL of CH₂Cl₂ was added Et₃N (0.1 mL). The mixture was cooled to -78°*C*, and then a solution of the acid chloride in CH₂Cl₂ was added. The reaction mixture was stirred at -78°*C* for 1h, and then allowed to warm to room temperature and stirred overnight. The solvent was evaporated and the residue was chromatographed on silica gel with PE/EtOAc (3:1 V/V) to give 44 (48 mg) as a white solid.
¹H NMR (CDCl₃, 300 MHz): δ (ppm) 8.55 (dd, J = 4.2,1.5 Hz, 1H), 8.25 (d, *J =* 16 Hz, 1H), 7.69-7.59 (m, 3H), 7.51 (d, *J=* 3.6 Hz, 1H), 7.40 (dt, *J =* 8.0,1.5 Hz, 1H), 7.03-6.94 (q, *J =* 8.0,18 Hz, 2H), 6.99 (d, *J =* 3.6 Hz, 1 H), 6.89 (d, *J =* 3.6 Hz, 1H);
¹³C NMR (CDCl₃, 300 MHz): δ (ppm) 165.67 (1C), 160.45 (1C), 158.89 (1C), 157.80 (1C), 148.49 (1C), 145.75 (1CH), 143.47 (1CH), 139.98 (1C), 138.09 (1CH), 133.86 (1CH), 132.35 (1CH), 129.76 (1CH), 128.62 (1CH), 123.16 (1C), 120.91 (1CH), 116.93 (1CH), 113.75(1CH), 111.38 (1CH), 55.67 (1CH₃);
EIMS (m/z): 381(M⁺, 7%), 353 (8), 324 (16), 225 (52), 221 (26), 161 (90), 127 (28), 123 (47), 95 (49), 71 (60), 69 (100).

### 8. Preparation of compound 47

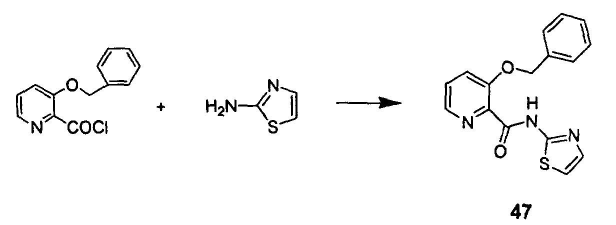

To a solution of the acid chloride in 2 mL of CH₂Cl₂ was added thiazol-2-ylamine (11.4mg, 0.114 mmol) and Et₃N (19 µL, 0.114 mmol) under argon atmosphere. The reaction mixture was stirred overnight, and then diluted with 10 mL of CH₂Cl₂, and washed with H₂O (3x30 mL), saturated aqueous NaCl (3 mL) and dried over anhydrous MgSO₄. The solvent was filtered ,evaporated and the residue was dissolved in CH₂Cl₂ and chromatographed on silica gel with PE/EtOAc (1:1 V/V) to give 6mg of 47 as a red solid.
¹H NMR (CDCl₃, 300MHz): δ (ppm) 8.25 (dd, *J* = 2.7,2.7 Hz, 1H), 7.52-7.50 (m, 3H), 7.42-7.32 (m, 5H), 7.02 (d, *J*= 3.6 Hz, 1H);
¹³C NMR (CDCl₃, 300MHz): δ (ppm) 161.30 (1C), 158.38 (1C), 156.33 (1C), 140.73 (1CH), 137.80 (1CH), 136.47 (1C), 135.85 (1C), 129.06 (2CH), 128.54 (1CH), 128.42 (1CH), 127.12 (1CH), 127.06 (1CH), 123.50 (1CH), 113.64 (1CH), 71.14 (1CH₂);
EIMS (m/z): 311 (M+, 6%), 220 (7), 197 (8), 189 (43), 123 (19), 111 (29), 97 (44), 91(100), 85 (55), 71 (83), 69 (78).

### 9. Preparation of compound 51

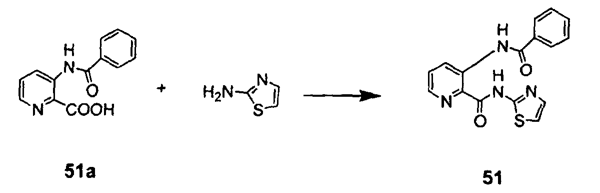

A mixture of 51a (40 mg, 0.165 mmol), DCC (41 mg, 0.198 mmol), DMAP (10 mg, 0.083 mmol, 0.5eq), 4ÅMS (100 mg) in dry toluene (1 mL) was stirred at room temperature for 20min under argon atmosphere, and then thiazol-2-ylamine (17 mg, 0.165 mmol) was added. The reaction mixture was stirred at 70°*C* for 1.5h and was then filtered. The filtrate was directly chromatographed on silica gel with PE/EtOAc (3:1 V/V) to give a white solid (4 mg).
¹H NMR (CDCl₃, 300MHz): δ (ppm) 9.40 (dd, *J* = 8.4,1.5Hz, 1H), 8.36 (dd, *J* = 4.5Hz, 1H), 8.12-8.09 (m, 2H), 7.62-7.55 (m, 5H), 7.09 (d, *J* = 3.6 Hz, 1H); EIMS (m/z): 324 (M+, 7), 239 (6), 225 (19), 197 (11), 125 (25), 121 (11), 111(39), 97 (60), 85 (65), 71 (100), 69 (96).

### 10. Preparation of compound 53

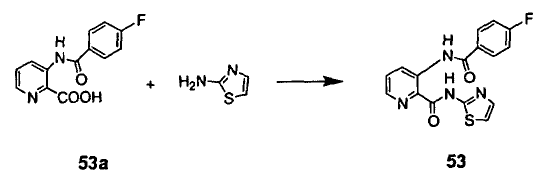

A mixture of 53a (75 mg, 0.288 mmol), DCC (77 mg, 0.375 mmol, 1.3eq), DMAP (18 mg, 0.144 mmol, 0.5eq), 4ÅMS (100 mg) in dry toluene (1 mL) was stirred at room temperature for 20min, and then thiazol-2-ylamine (29 mg, 0.288 mmol) was added. The reaction mixture was stirred at 70°*C* for 3h and was then filtered. The filtrate was directly chromatographed on silica gel with PE/EtOAc (1:1 V/V) to give 53 as a white solid (13 mg).
¹H NMR (CDCl₃, 300 MHz): δ (ppm) 12.49 (s, 1H), 11.38 (br, 1H), 9.36 (dd, *J* = 8.4,0.9 Hz, 1H), 8.36(dd, *J*= 4.5,0.9 Hz, 1H), 8.14-8.10 (m, 2H), 7.61-7.57 (m, 2H), 7.28-7.22 (m, 2H), 7.10 (d, *J*= 3.6Hz, 1H);
¹³C NMR (CDCl₃, 300MHz): δ (ppm) 167.27 (1C), 165.53 (1C), 165.12 (1C), 163.91 (1C), 157.06 (1C), 142.79 (1CH), 139.47 (1C), 138.62 (1CH), 131.52 (1C), 130.33 (1CH), 130.21 (1CH), 129.17 (1CH), 129.04 (1CH), 116.45 (1CH), 116.16 (1CH), 114.46 (1CH).

### 11. Preparation of compounds 55-59

### General procedure for the preparation of compounds 55-59:

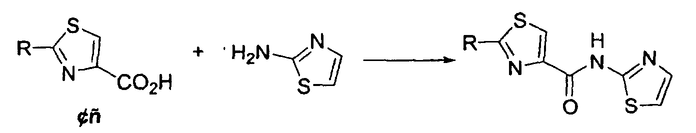

A mixture of *I*(1mmol), thiazol-2-ylamine (120 mg,1.2 mmol), HOBT(162 mg, 1.2 mmol), EDC (230 mg, 1.2 mmol) and little 4ÅMS in dry DMF (10mL) was stirred at room temperature overnight. The mixture was diluted with EtOAc and washed with H₂O for three times in order to remove DMF, then washed with saturated aqueous NaCl and dried over anhydrous MgSO₄. The solvent was evaporated under reduced pressure and chromatography afforded products 55-59. 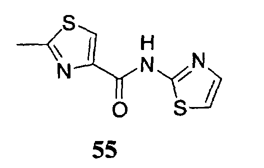 Compound 55: ¹H NMR (CDCl₃, 300 MHz): δ (ppm) 11.08 (br, 1H), 8.12 (s, 1H), 7.53(d, *J* = 3.6Hz, 1H), 7.00 (d, *J* = 3.6Hz, 1H), 2.70 (s, 3H); ¹³C NMR (CDCl₃, 300MHz): δ (ppm) 166.87 (1C), 158.54 (1C), 158.04 (1C), 147.70 (1C), 138.30 (1CH), 125.59 (1CH), 113.79 (1CH), 19.25 (1CH₃). 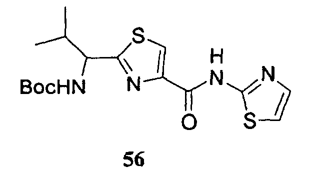 Compound 56: ¹H NMR (CDCl₃, 300MHz): δ (ppm) 10.64 (br, 1H), 8.20 (s, 1H), 7.49 (d, *J=* 3.6Hz, 1H), 7.02 (d, *J*= 3.6Hz, 1H), 5.30 (d, *J*= 8.7Hz, 1H), 4.89(m, 1H), 2.32 (m, 1H), 0.97 (d, *J* = 6.9Hz, 1H), 0.91 (d, *J* = 6.9Hz, 1H); ¹³C NMR (CDCl₃, 300MHz): δ (ppm) 173.84 (1C), 158.31 (1C), 157.78 (1C), 155.55 (1C), 147.97 (1C), 138.10 (1CH), 125.36 (1CH), 114.14 (1CH), 80.52 (1C), 58.10 (1CH), 33.39 (1CH), 28.44 (3CH₃), 19.41 (1CH₃), 17.59 (1CH₃). 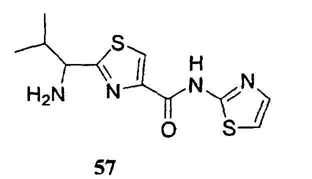 Compound 57: ¹H NMR (CDCl₃, 300MHz): δ (ppm) 8.66 (br, 2H), 7.63 (d, *J* = 3.9Hz, 1H), 7.21 (d, *J* = 3.9Hz, 1H), 4.86 (m, 1H), 2.50 (m, 1H), 1.11 (d, *J* = 6.9Hz, 1H), 0.98 (d, *J* = 6.9Hz, 1H). 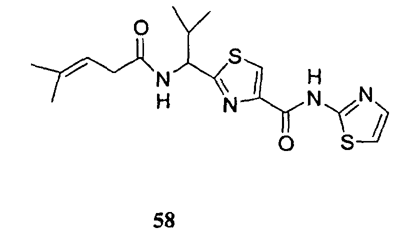 Compound 58: ¹H NMR (CDCl₃, 300MHz): δ (ppm) 8.22 (s, 1H), 7.51 (d, *J* = 3.6Hz, 1H), 7.05 (d, *J* = 3.6Hz, 1H), 6.60 (d, *J*= 8.7Hz, 1H), 5.38 (m, 1H), 5.24 (dd, *J* = 8.7,5.7Hz, 1H), 3.06 (d, *J* = 7.8Hz, 2H), 2.38 (m, 1H), 1.82 (s, 3H), 1.69 (s, 3H), 0.88 (d, *J* = 6.9Hz, 3H), 0.86 (d, *J* = 6.9Hz, 3H). 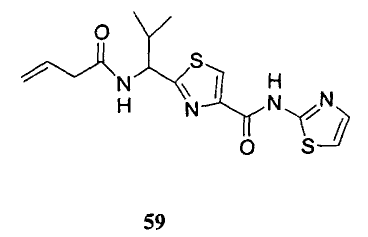 Compound 59: ¹H NMR (CDCl₃, 300MHz): δ (ppm) 8.22 (s, 1H), 7.50 (d, *J*= 3.6Hz, 1H), 7.05 (d, *J* = 3.6Hz, 1H), 6.77 (d, *J* = 8.7Hz, 1H), 6.00 (m, 1H), 5.26 (m, 3H), 3.12 (d, *J*= 6.9Hz, 2H), 2.33 (m, 1H), 0.97 (d, *J* = 6.6Hz, 3H), 0.96 (d, *J*= 6.6Hz, 3H)

## Claims

1. A methionine aminopeptidase inhibitor represented by the general formular 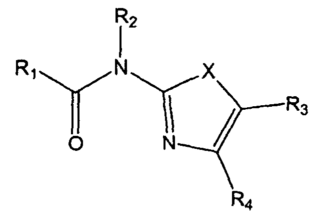 wherein
R₁ is selected from the group consisting of C₁-C₄ alkyl, substituted alkyl, C₃-C₆ cycloalkyl, substituted cycloalkyl, aryl, pyridyl; substituted aryl and substituted pyridyl wherein the substituents can be optionally selected from the group consisting of C₁-C₄ alkyl, nitro, carboxyl, aldehyde, alkoxy, alkylamino, acylamide, alkylthio; heterocycle or substituted heterocycle having the following structure: 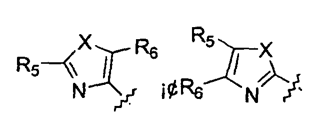 R₅, R₆ are selected from the group consisting of hydrogen, C₁-C₄ alkyl, substituted alkyl, C₃-C₆ cycloalkyl, substituted cycloalkyl, aryl, pyridyl; substituted aryl and substituted pyridyl wherein the substituents can be optionally selected from the group consisting of nitro, carboxyl, aldehyde, alkoxy, alkylamino, acylamide, alkylthio;
R₂ is selected from the group consisting of hydrogen, C₁-C₄ alkyl, substituted alkyl, aryl, substituted aryl wherein the substituents can be optionally selected from the group consisting of C₁-C₄ alkyl, nitro, carboxyl, aldehyde, alkoxy, alkylamino, acylamide, alkylthio;
R₃ is selected from the group consisting of hydrogen, C₁-C₄ alkyl, substituted C₁-C₄ alkyl, halogen atoms; aryl, substituted aryl;
R₄ is selected from the group consisting of hydrogen, C₁-C₄ alkyl, substituted alkyl, substituted aryl;
X is selected from the group consisting of O, S, N.

2. A methionine aminopeptidase inhibitor according to claim 1 in which R₁ is selected from the group consisting of pyridyl, substituted pyridyl wherein the substituents can be optionally selected from the group consisting of halogen atoms, acylamide, alkoxy, hydroxyl, carboxyl, alkoxycarbonyl, ether;
R₂ is hydrogen;
R₃ is selected from the group consisting of hydrogen, Br, alkyl;
R₄ is selected from the group consisting of hydrogen, alkyl, substituted aryl;

3. A methionine aminopeptidase inhibitor according to claim 1 in which R₁ is selected from the group consisting of aryl, substituted aryl wherein the substituents can be optionally selected from the group consisting of nitro, alkylamino, C₁-C₄ alkoxy, hydroxyl, carboxyl, benzyl;
R₂ is hydrogen;
R₃ is selected from the group consisting of hydrogen, halogen atoms, C₁-C₄ alkyl;
R₄ is selected from the group consisting of hydrogen, C₁-C₄ alkyl, substituted aryl;

4. A methionine aminopeptidase inhibitor according to claim 1 in which R₁ is selected from the group consisting of heterocycle, substituted heterocycle;
R₂ is hydrogen;
R₃ is hydrogen;
R₄ is hydrogen;
R₅, R₆ are selected from the group consisting of hydrogen, C₁-C₄ alkyl, substituted alkyl, C₃-C₆ cycloalkyl, substituted cycloalkyl, aryl, pyridyl; substituted aryl and substituted pyridyl wherein the substituents can be optionally selected from the group consisting of nitro, carboxyl, aldehyde, alkoxy, alkylamino, acylamide, alkylthio;

5. A process for the preparation of a methionine aminopeptidase inhibitor as defined in claim 1 which comprises condensating of a compound of the general formula R1COY with a compound of the general formula 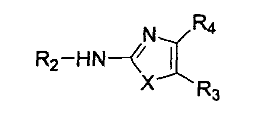 in which Y represents hydroxyl, halogen atoms and the other activated group;

6. A process for the preparation of a methionine aminopeptidase inhibitor as defined in claim 4 wherein the dehydration reagents used in this reaction may be DCC, ECD, DIC, HBTU;

7. A process for the preparation of a methionine aminopeptidase inhibitor as defined in claim 4 wherein the solvent used in this condensation reaction may be CH₂Cl₂, DMF, CH₂ClCH₂Cl, toluene, benzene, H₂O, dioxane or the mixture of the above solvents;

8. A process for the preparation of a methionine aminopeptidase inhibitor as defined in claim 4 wherein the reaction temperature is from -20°C to room temperature, in some cases, the heating is necessary, from 50° C to 130°C;

9. A process for the preparation of a methionine aminopeptidase inhibitor as defined in claim 4 wherein the proper activated reagents of the condensation reaction were used, such as, HOBT pentafluorophenol, molecular series;

10. A process for the preparation of a methionine aminopeptidase inhibitor as defined in claim 4 wherein the proper base of the condensation reaction such as Et₃N, I-Pr₂EtN, Pyridine, DMAP were used as catalyst;

11. A methionine aminopeptidase inhibitor as claimed in claim 1, wherein these compounds were used as antitumor, and anti-infection lead compounds.
